# EUROPEAN PATENT APPLICATION

(11) **EP 3 576 043 A1**
(43) Date of publication of application: **04.12.2019**
(21) Application number: 18754204.8
(22) Date of filing: 15.02.2018
(51) Int. Cl.: G06Q 50/22, G16H 10/00

(54) **MEDICAL INFORMATION MANAGEMENT SYSTEM, CLINICAL INFORMATION ACQUISITION SERVER, MEDICAL INFORMATION MANAGEMENT METHOD, AND PROGRAM**

(30) Priority: 15.02.2017 JP 2017026368
(71) Applicant: Foundation for Biomedical Research and Innovation at Kobe, Kobe-shi Hyogo 650-0047 (JP)
(72) Inventor: YUASA, Keisuke, Kobe-shi Hyogo 650-0047 (JP); SHIOYA, Masahiro, Kobe-shi Hyogo 650-0047 (JP); NAKAGAWA, Tomofumi, Kobe-shi Hyogo 650-0047 (JP); TAKENOUCHI, Kiyoteru, Kobe-shi Hyogo 650-0047 (JP)
(74) Representative: Prüfer & Partner mbB Patentanwälte · Rechtsanwälte
(86) International application number: PCT/JP2018/005149
(87) International publication number: WO 2018/151181

(57) **Abstract**

Provided is a medical information management system including: a standardized storage (231) that stores, in a single format, a plurality of pieces of electronic patient record information that is stored in a plurality of electronic patient record servers storing the plurality of pieces of electronic patient record information in different formats; a definition information generator that generates definition information that defines a combination of search keys for retrieving a piece of electronic patient record information on a specific case from among the plurality of pieces of electronic patient record information; a potential case retriever (214) that acquires, based on the definition information, a piece of electronic patient record information on the specific case from among the plurality of pieces of electronic patient record information; and a test value information extractor (515) that extracts test value information, which is clinical information of a patient associated with the specific case from the piece of electronic patient record information acquired by the potential case retriever (214).

## Description

### Technical Field

The present disclosure relates to a medical information management system, a clinical information acquisition server, a medical information management method, and a program.

### Background Art

There has been proposed a data collection and processing system that includes a terminal and a clinical trial data collection server communicably connected with the terminal (see, for example, Patent Literature 1). The clinical trial data collection server accumulates electronic patient record information containing information on prescribed medicines, diagnoses, and test values acquired at medical institutions.

### Citation List

### Patent Literature

Patent Literature 1: Unexamined Japanese Patent Application Kokai Publication No. 2008-276761.

### Summary of Invention

### Technical Problem

Incidentally, assume a plurality of clinical trial data collection servers that is provided by vendors different from each other and accumulate a plurality of pieces of electronic patient record information in formats incompatible with each other. When a user tries to collect specific clinical information from these clinical trial data collection servers, the user is compelled, with respect to test value information contained in respective pieces of electronic patient record information accumulated in the plurality of clinical trial data collection server, to check the electronic patient record information in each clinical trial data collection server and read the clinical information contained in the electronic patient record information and to store the test value information thus read in a non-transitory recording medium. In this case, the efficiency in collecting the clinical information contained in the electronic patient record information turns out low.

The present disclosure is made in view of the above-described situation and an objective of the present disclosure is to provide a medical information management system, a clinical information acquisition server, a medical information management method, and a program that enable efficient collection of clinical information.

### Solution to Problem

To achieve the above-described objective, a medical information management system according to the present disclosure includes:
an electronic patient record information storage that stores a plurality of pieces of electronic patient record information in a single format, the plurality of pieces of electronic patient record information being stored in a plurality of electronic patient record servers storing the plurality of pieces of electronic patient record information in different formats;
a definition information generator that generates definition information that defines a combination of search keys for retrieving a piece of electronic patient record information on a specific case from among the plurality of pieces of electronic patient record information;
an electronic patient record information retriever that acquires, based on the definition information, a piece of electronic patient record information on the specific case from among the plurality of pieces of electronic patient record information; and
a clinical information extractor that extracts a piece of clinical information of a patient associated with the specific case from the piece of electronic patient record information acquired by the electronic patient record information retriever.

A clinical information acquisition server according to the present disclosure in another aspect includes
a clinical information extractor that extracts a piece of clinical information of a patient associated with a specific case from a piece of electronic patient record information on the specific case acquired from among a plurality of pieces of electronic patient record information, based on definition information that defines a combination of search keys for retrieving the piece of electronic patient record information on the specific case from among the plurality of pieces of electronic patient record information that is stored in an electronic patient record information storage.

A medical information management method according to the present disclosure in another aspect is
a medical information management method using an electronic patient record information storage that stores a plurality of pieces of electronic patient record information in a single format, the plurality of pieces of electronic patient record information being stored in a plurality of electronic patient record servers storing the plurality of pieces of electronic patient record information in different formats, the method including the steps of:
generating definition information that defines a combination of search keys for retrieving a piece of electronic patient record information on a specific case from among the plurality of pieces of electronic patient record information;
acquiring, based on the definition information, a piece of electronic patient record information on the specific case from among the plurality of pieces of electronic patient record information; and
extracting a piece of clinical information of a patient associated with the specific case from the acquired piece of electronic patient record information.

A program according to the present disclosure in another aspect causes a computer to function as:
an electronic patient record information storage that stores a plurality of pieces of electronic patient record information in a single format, the plurality of pieces of electronic patient record information being stored in a plurality of electronic patient record servers storing the plurality of pieces of electronic patient record information in different formats;
a definition information generator that generates definition information that defines a combination of search keys for retrieving a piece of electronic patient record information on a specific case from among the plurality of pieces of electronic patient record information;
an electronic patient record information retriever that acquires, based on the definition information, a piece of electronic patient record information on the specific case from among the plurality of pieces of electronic patient record information; and
a clinical information extractor that extracts a piece of clinical information of a patient associated with the specific case from the piece of electronic patient record information acquired by the electronic patient record information retriever.

### Advantageous Effects of Invention

According to the present disclosure, an electronic patient record information storage stores, in a single format, a plurality of pieces of electronic patient record information that is stored in a plurality of electronic patient record servers storing electronic patient record information in different formats. An electronic patient record information retriever then acquires a piece of electronic patient record information on a specific case from among the plurality of pieces of electronic patient record information on the basis of definition information generated by a definition information generator, and a clinical information extractor extracts a piece of clinical information of a patient associated with the specific case from the piece of electronic patient record information acquired by the electronic patient record information retriever. This allows the collection of a plurality of pieces of the clinical information simply by accessing the electronic patient record information storage as if separately collecting, from the plurality of pieces of electronic patient record information accumulated in each of the plurality of electronic patient record servers, a plurality of pieces of the clinical information contained respectively in the plurality of pieces of electronic patient record information. It is therefore possible to collect efficiently the plurality of pieces of clinical information contained respectively in the plurality of pieces of electronic patient record information accumulated in the above-described plurality of electronic patient record servers. Further, there are other advantages such as, for example, the possibility of facilitating the promotion of secondary uses of the collected pieces of clinical information.

### Brief Description of Drawings

FIG. 1 is a schematic block diagram of a medical information management system according to an embodiment of the present disclosure;
FIG. 2 is a block diagram illustrating a terminal device according to the embodiment;
FIG. 3 is a diagram illustrating contents of a table stored in a definition information memory according to the embodiment;
FIG. 4 is a block diagram illustrating a storage server, a test value information acquisition server, and an electronic patient record server according to the embodiment;
FIG. 5 is a diagram illustrating a directory structure of a standardized storage according to the embodiment;
FIG. 6 is a diagram illustrating contents of a table stored in a test value information memory according to the embodiment;
FIG. 7 is a sequence diagram illustrating an example operation of the terminal device, the test value information acquisition server, and the storage server according to the embodiment;
FIG. 8 is a diagram illustrating an example of an operation screen displayed on a display device of the user interface of the storage server according to the embodiment;
FIG. 9 is a sequence diagram illustrating an example operation of the storage server and the electronic patient record server according to the embodiment;
FIG. 10 is a flow chart illustrating an example flow of test value information acquisition processing executed by the terminal device according to the embodiment;
FIG. 11 is a flow chart illustrating an example flow of test value information transmission processing executed by the test value information acquisition server according to the embodiment;
FIG. 12 is a flow chart illustrating an example flow of electronic patient record information retrieval processing executed by the storage server according to the embodiment;
FIG. 13 is a flow chart illustrating an example flow of standardized storage management processing executed by the storage server according to the embodiment;
FIG. 14 is a flow chart illustrating an example flow of electronic patient record information transmission processing executed by the electronic patient record server according to the embodiment;
FIG. 15 is a block diagram illustrating a configuration of a terminal device according to a modified example;
FIG. 16A is a diagram illustrating contents of a table stored in a determination standard memory according to the modified example;
FIG. 16B is a diagram illustrating contents of a table stored in a PPV memory according to the modified example; and
FIG. 17 is a flow chart illustrating an example flow of PPV evaluation processing executed by the terminal device according to the modified example.

### Description of Embodiments

A medical information management system according to an embodiment of the present disclosure will be described below in detail with reference to the drawings. The medical information management system according to the present embodiment includes a storage server that accumulates electronic patient record information and acquires a specific piece of electronic patient record information from among the pieces of the electronic patient record information accumulated in the storage server by using definition information that defines a combination of search keys. The medical information management system then acquires a desired piece of clinical information from the acquired piece of electronic patient record information.

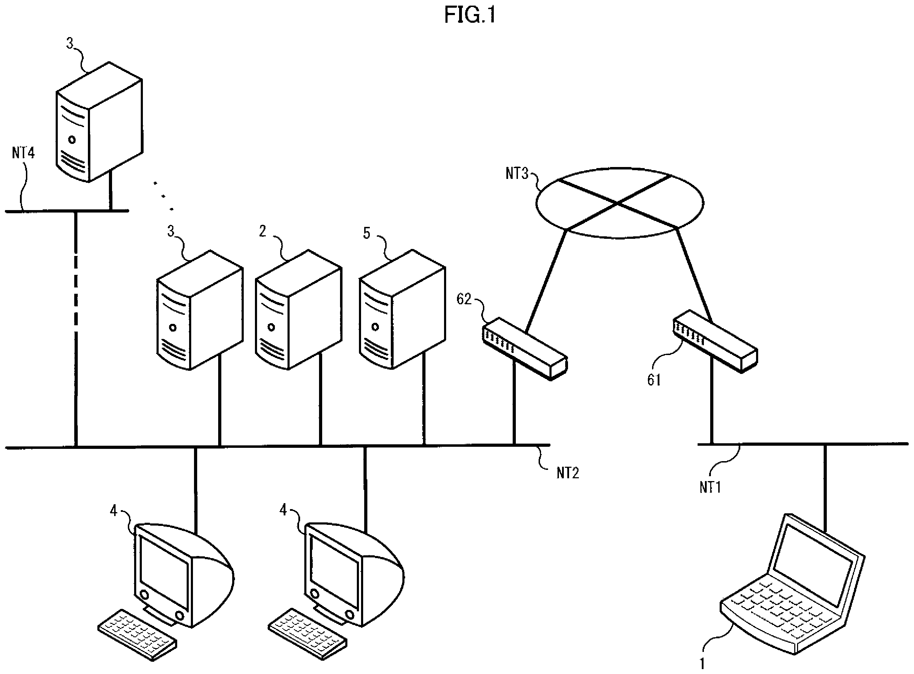

The medical information management system according to the present embodiment includes, as illustrated in FIG. 1, a terminal device 1 installed in a laboratory where clinical tests of medicines are conducted, a test value information acquisition server (clinical information acquisition server) 5 installed in a medical facility, a storage server 2, and a plurality of electronic patient record servers 3. The terminal device 1 is connected to a network NT1 installed in the laboratory. The test value information acquisition server 5 and the storage server 2 are connected to a network NT2 installed in the medical facility. Further, the plurality of electronic patient record servers 3 is connected to the network NT2 installed in the medical facility or to a network NT4, which is different from the network NT2. The plurality of electronic patient record servers 3 is provided by vendors different from each other and store a plurality of pieces of electronic patient record information in formats different from each other. Further, terminal devices 4 used by users in the medical facility (for example, doctors and pharmacists) are connected to the network NT2. The network NT1 of the laboratory and the network NT2 of the medical facility are connected to a wide area network NT3 via firewall devices 61, 62, respectively. A user in the laboratory inputs, for example, to the terminal device 1, a combination of search keys for extracting test value information of a test conducted on a patient who suffers a specific disease. The terminal device 1 then generates definition information that defines the inputted combination of search keys and transmits the generated definition information to the storage server 2 in the medical facility. Upon receiving the definition information, the test value information acquisition server 5 in turn transmits the received definition information to the storage server 2. The storage server 2 retrieves electronic patient record information, using the combination of search keys defined by the received definition information, and transmits the electronic patient record information thus obtained to the test value information acquisition server 5. The test value information acquisition server 5 extracts test value information from the received electronic patient record information and transmits the extracted test value information to the terminal device 1 in the laboratory. Upon receiving the test value information from the test value information acquisition server 5, the terminal device 1 stores the received test value information.

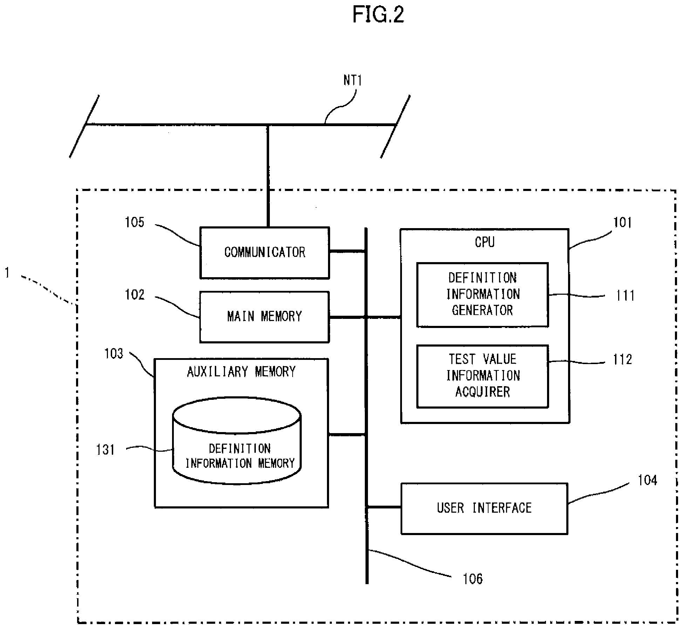

The terminal device 1 includes, for example, a general-purpose personal computer and, as illustrated in FIG. 2, includes a central processing unit (CPU) 101, a main memory 102, an auxiliary memory 103, a user interface 104, a communicator 105, and a bus 106 that connects these mutually. The main memory 102 includes a volatile memory, used as a working area for the CPU 101. The auxiliary memory 103 includes a non-volatile memory such as a magnetic disc or a semiconductor memory and stores a program for realizing the various functions of the terminal device 1. The various functions of the terminal device 1 are then realized by the CPU 101 reading out the program into the main memory 102 and executing the program. The communicator 105 is connected to the network NT1 installed in the laboratory. The user interface 104 includes an input device such as, for example, a key board or a touch panel and a display device including a display.

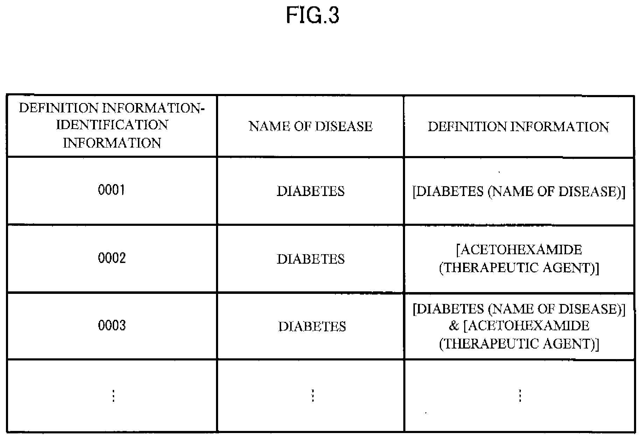

The CPU 101 functions as a definition information generator 111 and a test value information acquirer 112 by executing the program stored in the auxiliary memory 103. Further, the auxiliary memory 103 includes a definition information memory 131. As illustrated in FIG. 3, for example, the definition information memory 131 stores a table on which pieces of definition information defining combinations of search keys are associated with the names of the diseases corresponding to the pieces of definition information and with the pieces of definition information-identification information.

Referring to FIG. 2 again, the definition information generator 111 generates definition information that defines a combination of search keys for retrieving a piece of electronic patient record information on a specific case (to be referred to as "potential case" hereinafter) from among a plurality of pieces of electronic patient record information stored in a standardized storage, which will be described later. A search key may be information indicating a name of a disease, a therapeutic agent, an act of medical care included in the electronic patient record information. A potential case is a case that corresponds to the electronic patient record information mechanically extracted based on the definition information. The definition information is generated from the combination of search keys inputted by the user to the terminal device 1 via the user interface 104 for extracting the test value information that indicates the result of a medical test conducted on a patient who suffers the specific disease. The definition information generator 111 then stores the generated definition information in the definition information memory 131, with definition information-identification information added to the definition information. The definition information is stored in the definition information memory 131 in the form of, for example, an XML file.

By acquiring the definition information from the definition information memory 131 and sending the definition information to the test value information acquisition server 5, the test value information acquirer 112 acquires test value information for each of the patients who correspond to the search keys in the definition information (to be referred to as "potential cases" hereinafter) from the test value information acquisition server 5.

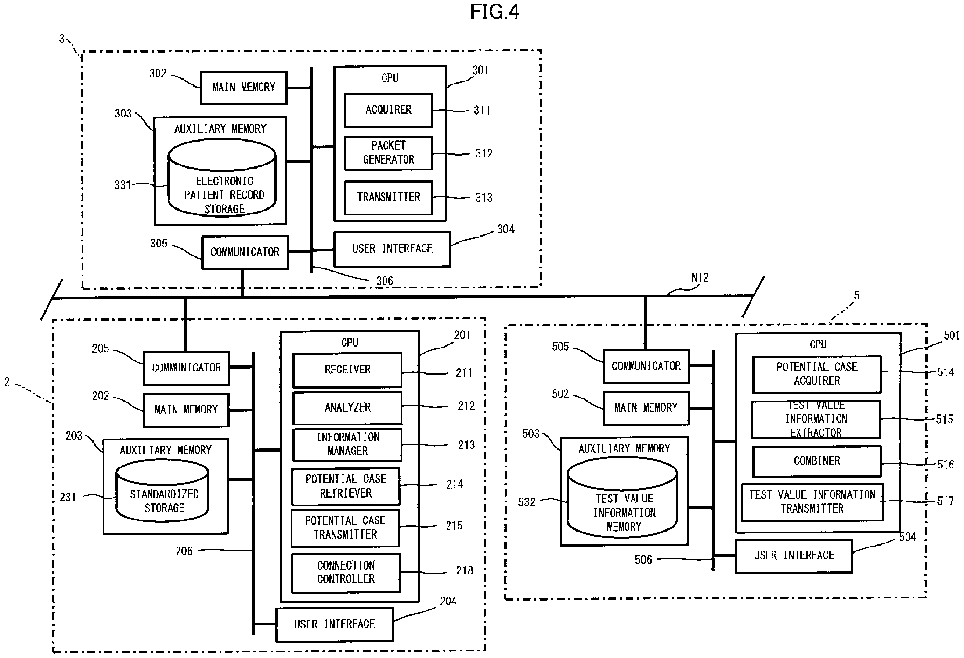

As illustrated in FIG. 4, the storage server 2 includes a CPU 201, a main memory 202, an auxiliary memory 203, a user interface 204, a communicator 205, and a bus 206 that connects these mutually. The auxiliary memory 203 stores a program for realizing the various functions of the storage server 2. The various functions of the storage server 2 are then realized by the CPU 201 reading out the program into the main memory 202 and executing the program. The communicator 205 is connected to the network NT2 installed in the medical facility.

The CPU 201 functions as a receiver 211, an analyzer 212, an information manager 213, a potential case retriever 214, and a connection controller 218 by executing the program stored in the auxiliary memory 203. The auxiliary memory 203 includes a standardized storage (electronic patient record information storage) 231.

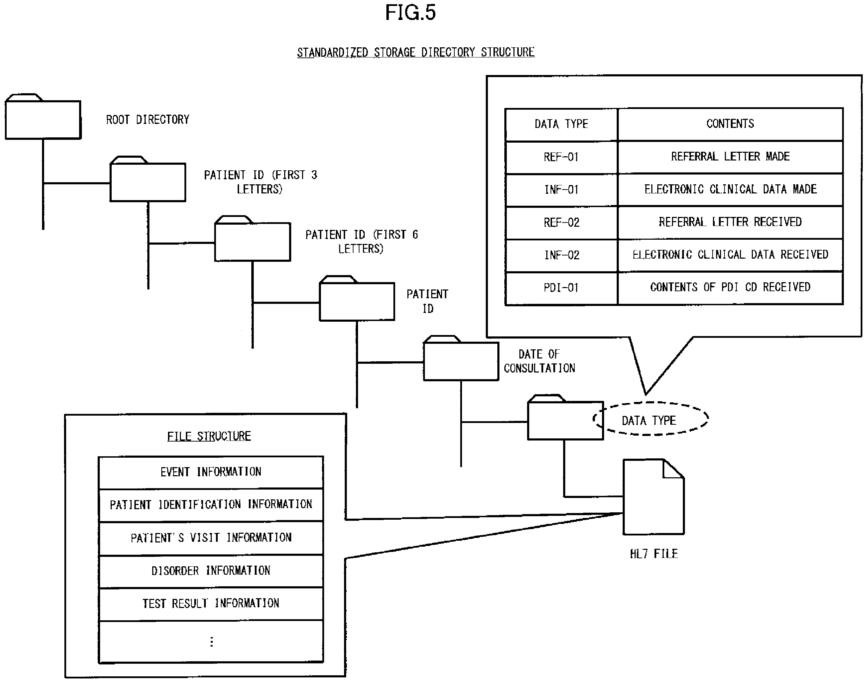

The standardized storage 231 includes, for example, an SS-MIX standardized storage and stores, in a single format, electronic patient record information that is stored in a plurality of electronic patient record servers 3 in different formats. The single format may be, for example, a format adapted to Health Level 7 (HL7). Here, HL refers to standards for a comprehensive framework of electronic heath care information. The name of each file has a structure of, for example, "(patient identification information) _(date of consultation) (data type)_(order number)_(time and date of occurrence)_(department)_(condition flag)". Here, the data type includes information on the exchanges of medical information, as illustrated in a balloon in FIG. 5. The order number is an identification number for identifying an instruction by doctors, nurses, or the like. The time and date of occurrence indicates the time and the date when the file is created. The condition flag is a piece of flag information indicating whether or not the file is valid. As illustrated in a balloon in FIG. 5, the file structure includes event information, patient identification information, information on the patient's visit to the medical facility, disorder information, and test result information. Here, the event information is information indicating the date of the patient's visit to the medical facility, the date of occurrence of the disease, and the like. The patient identification information is information indicating the patient's name, address, and the like. The information on the patient's visit to the medical facility is information indicating the name of a person who accompanied the patient, the number of times of the visits to the medical facility. The disorder information is information indicating the abnormality observed in the patient. The test result information is information indicating the result of a test conducted on the patient.

Further, for example, as illustrated in FIG. 5, the standardized storage 231 manages the electronic patient record information in a directory structure according to the patient identification information, the information indicating the date of consultation, and the information indicating the data type included in each of a plurality of pieces of electronic patient record information.

Referring to FIG. 4 again, the receiver 211 receives the definition information transmitted by the terminal device 1 in the laboratory and an electronic patient record information packet transmitted by the electronic patient record server 3. The analyzer 212 analyzes the electronic patient record information packet received by the receiver 211 and extracts the patient identification information, the information indicating the date of consultation, and the information indicating the data type contained in the electronic patient record information packet.

The information manager 213 sets a file path for the acquired electronic patient record information on the basis of the patient identification information, the information indicating the date of consultation, and the information indicating the data type contained in the electronic patient record information. The information manager 213 then stores the electronic patient record information in the standardized storage 231 on the basis of the file path thus set.

The potential case retriever 214 extracts definition information from an electronic patient record information acquisition request and, on the basis of the extracted definition information, searches for and acquires a piece of electronic patient record information on the potential case from among a plurality of pieces of electronic patient record information.

The potential case transmitter 215 transmits the electronic patient record information searched for and acquired by the potential case retriever 214 to the test value information acquisition server 5.

The connection controller 218 executes authentication processing for the electronic patient record server 3, which is the transmission origin of the connection request. Further, when the authentication of the electronic patient record server 3 is successful, the connection controller 218 establishes a connection with the electronic patient record server 3 and transmits a connection approval notification to the electronic patient record server 3.

The test value information acquisition server 5 includes, as illustrated in FIG. 4, a CPU 501, a main memory 502, an auxiliary memory 503, a user interface 504, a communicator 505 and a bus 506 that connects these mutually. The auxiliary memory 503 stores a program for realizing the various functions of the test value information acquisition server 5. The various functions of the test value information acquisition server 5 are then realized by the CPU 501 reading out the program into the main memory 502 and executing the program. The communicator 505 is connected to the network NT2 installed in the medical facility.

The CPU 501 functions as a potential case acquirer (electronic patient record information retriever) 514, a test value information extractor (clinical information extractor) 515, a combiner 516, and a test value information transmitter 517 by executing the program stored in the auxiliary memory 503. The auxiliary memory 503 includes a test value information memory 532.

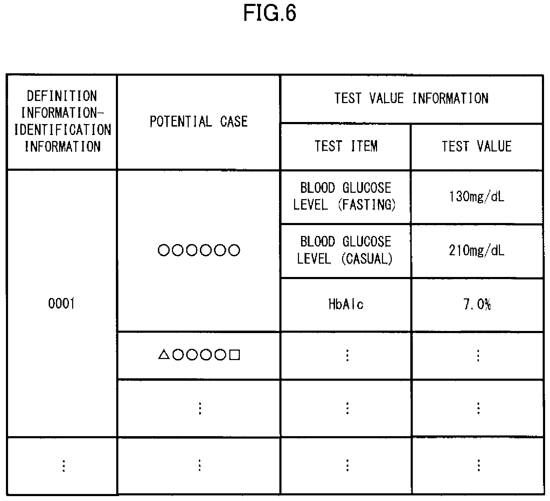

As illustrated in FIG. 6, the test value information memory 532 stores a table on which pieces of test value information corresponding to pieces of definition information are associated with pieces of definition information-identification information and potential cases. The test value information includes test items and respective test values of the test items. Further, the test value information memory 532 also stores test value information obtained by combining a plurality of pieces of test value information corresponding to one piece of definition information. The test value information is stored in the test value information memory 532 in the form of, for example, an XML-file.

Referring to FIG. 4 again, the potential case acquirer 514 generates an electronic patient record information request containing the definition information received from the terminal device 1 in the laboratory. By transmitting the generated electronic patient record information request to the storage server 2, the potential case acquirer 514 then acquires electronic patient record information of the potential case from the storage server 2.

The test value information extractor 515 extracts, from the electronic patient record information acquired by the potential case acquirer 514, test value information indicating the result of a medical test, which is clinical information of the patient associated with the potential case. The test value information extractor 515 then stores the extracted test value information in the test value information memory 532.

The combiner 516 combines a plurality of pieces of test value information in accordance with the combination condition stipulated by the user. When the user inputs information specifying a combination condition via the user interface 504 of the test value information acquisition server 5, the combiner 516 receives a test value information combination request, which is a request for combining a plurality of pieces of test value information. This test value information combination request contains information indicating the combination condition stipulated by the user. The combiner 516 then combines a plurality of test value information in accordance with the information contained in the test value information combination request and indicating the combination condition. Further, the combiner 516 compresses mutually combined pieces of test value information and stores the compressed information in the test value information memory 532.

The test value information transmitter 517 acquires the compressed test value information from the test value information memory 532 and transmits the acquired test value information to the terminal device 1.

The plurality of electronic patient record servers 3 stores a plurality of pieces of electronic patient record information in formats different from each other. Each electronic patient record server 3 includes a CPU 301, a main memory 302, an auxiliary memory 303, a user interface 304, a communicator 305, and a bus 306 that connects these mutually. The auxiliary memory 303 stores a program for realizing the various functions of the electronic patient record server 3. The various functions of the electronic patient record server 3 are then realized by the CPU 301 reading out the program into the main memory 302 and executing the program. The communicator 305 is connected to the network NT2 installed in the medical facility.

The CPU 301 functions as an acquirer 311, a packet generator 312, and a transmitter 313 by executing the program stored in the auxiliary memory 303. Further, the auxiliary memory 303 includes an electronic patient record storage 331.

The acquirer 311 acquires electronic patient record information from the terminal device 4 and stores the acquired electronic patient record information in the electronic patient record storage 331. The electronic patient record storage 331 stores a plurality of pieces of electronic patient record information in different, mutually incompatible formats. The packet generator 312 generates an electronic patient record information packet containing electronic patient record information stored in the electronic patient record storage 331. The transmitter 313 adds header information based on HL7 to the electronic patient record information, which has been converted by the packet generator 312 into a format adapted to HL7, and transmits the electronic patient record information to the storage server 2.

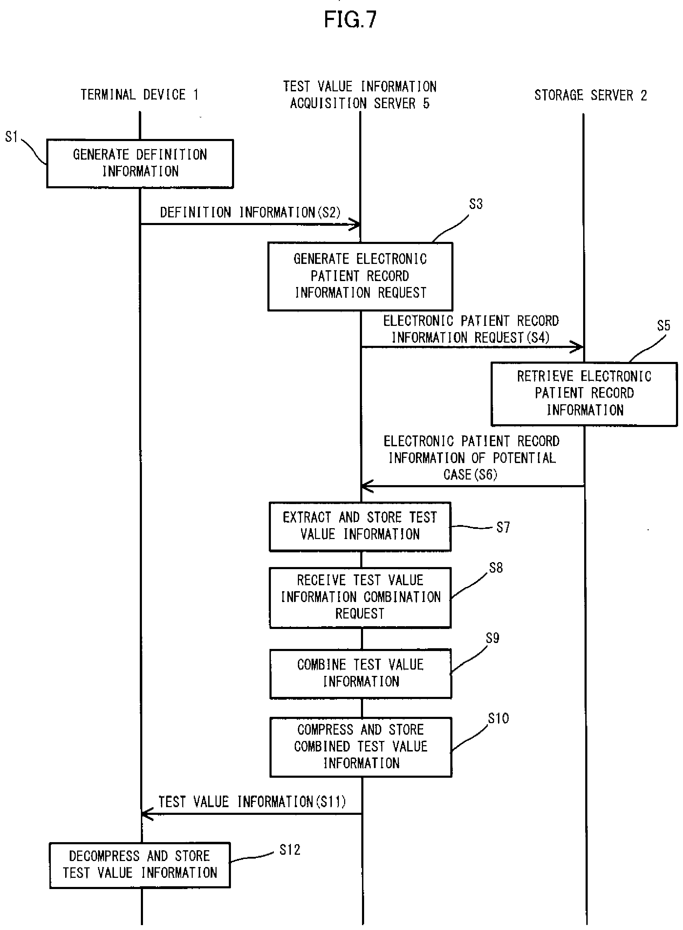

Next, an operation of the terminal device 1, the test value information acquisition server 5, and the storage server 2 will be described with reference to FIG. 7. First, when the user in the laboratory inputs a combination of search keys for retrieving a potential case to the terminal device 1 via the user interface 104, the terminal device 1 generates definition information from the inputted combination of the search keys (Step S1). Next, the generated definition information is transmitted from the terminal device 1 to the test value information acquisition server 5 (Step S2).

Upon receiving the definition information, the test value information acquisition server 5 in turn generates an electronic patient record information request that contains the definition information (Step S3). Subsequently, the electronic patient record information request is transmitted from the test value information acquisition server 5 to the storage server 2 (Step S4). Upon acquiring the electronic patient record information request from the test value information acquisition server 5, the storage server 2 in turn extracts the definition information contained in the acquired electronic patient record information request and, by using the definition information, searches the standardized storage 231 (Step S5) and acquires the electronic patient record information of the potential case. Thereafter, the acquired electronic patient record information of the potential case is transmitted from the storage server 2 to the test value information acquisition server 5 (Step S6). The test value information acquisition server 5 then extracts test value information from the electronic patient record information of the potential case received from the storage server 2 and stores the extracted test value information in the test value information memory 532 (Step S7).

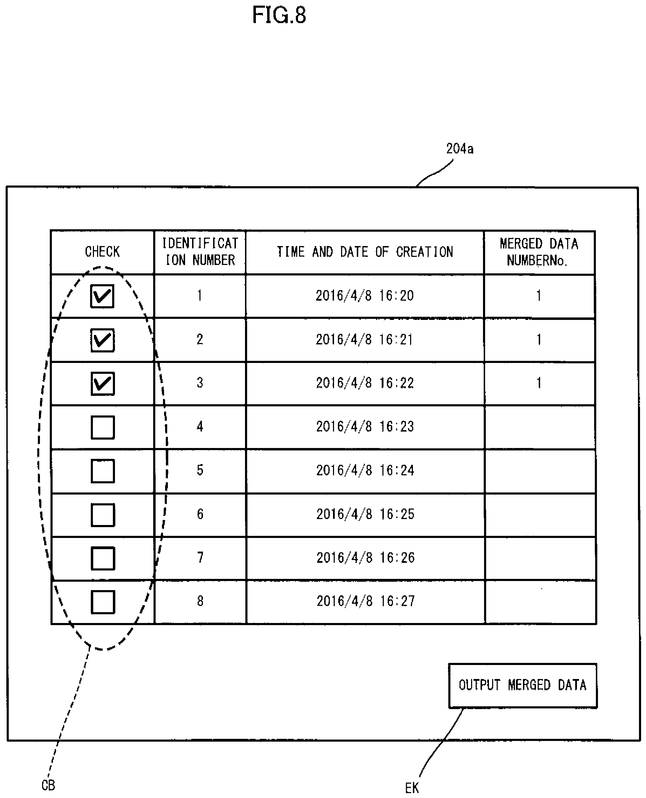

Next, assume that the user of the medical facility has made an operation on the test value information acquisition server 5 via the user interface 204 for combining pieces of test value information. In this case, the test value information acquisition server 5 receives a test value information combination request for combining pieces of test value information (Step S8). Here, an operation screen 204a, for example, as illustrated in FIG. 8 is displayed on the display device of the user interface 204. Assume that the user puts check marks in the check boxes beside the pieces of test value information (see the area CB, encircled by a broken line in FIG. 8) and clicks the merged data output button EK. In this case, the test value information acquisition server 5 receives a test value information combination request containing the combination condition for combining the pieces of test value information having identification numbers 1 to 3.

Referring to FIG. 7 again, upon receiving the test value information combination request, the test value information acquisition server 5 combines pieces of test value information in accordance with the combination condition specified by the user (Step S9). Subsequently, the test value information acquisition server 5 compresses the combined pieces of test value information and stores the compressed information in the test value information memory 532 (Step S10). Thereafter, the compressed test value information is transmitted from the test value information acquisition server 5 to the terminal device 1 (Step S11).

Upon receiving the test value information, the terminal device 1 in turn decompresses the received test value information and stores the decompressed information in the main memory 102 (Step S12).

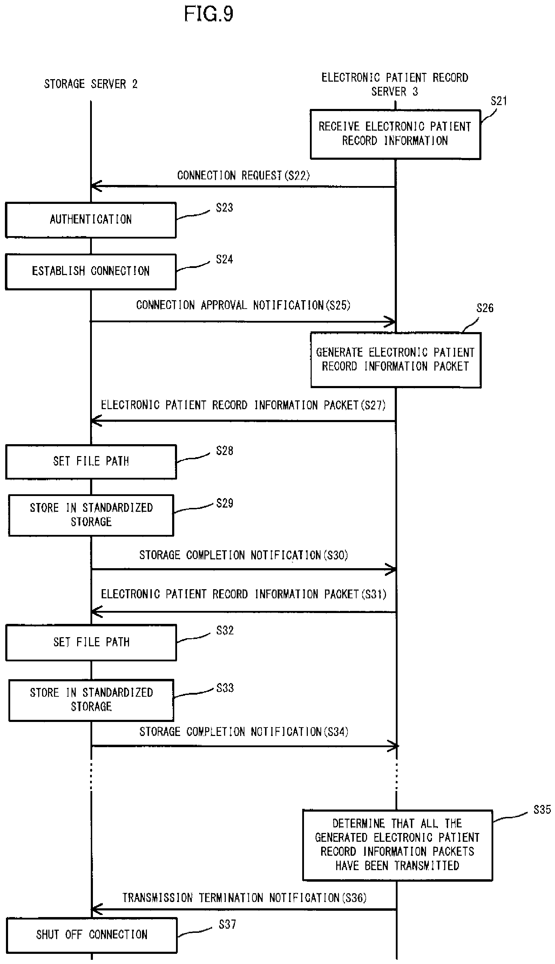

Next, an operation of the storage server 2 and the electronic patient record server 3 will be described with reference to FIG. 9. The storage server 2 executes an operation to be described below in parallel with the operation described above with reference to FIG. 7. First, when the user of the medical facility conducts the operation on a terminal device 4 for transmitting electronic patient record information to an electronic patient record server 3, the electronic patient record server 3 receives the electronic patient record information from the terminal device 4 (Step S21). At this time, a connection request for establishing a connection is transmitted from the electronic patient record server 3 to the storage server 2 (Step S22). Further, the electronic patient record server 3 stores the received electronic patient record information to the electronic patient record storage 331.

Upon receiving the connection request, the storage server 2 in turn executes authentication processing for the electronic patient record server 3, which is the transmission origin of the connection request (Step S23). Here, when the authentication of the electronic patient record server 3 is successful, the storage server 2 establishes the connection with the electronic patient record server 3 (Step S24). Next, a connection approval notification for notifying the electronic patient record server 3 of the approval for the connection with the storage server 2 is transmitted from the storage server 2 to the electronic patient record server 3 (Step S25).

Upon receiving the connection approval notification from the storage server 2, the electronic patient record server 3 acquires the electronic patient record information from the electronic patient record storage 331 and generates an electronic patient record information packet from the acquired electronic patient record information (Step S26). Here, the electronic patient record server 3 generates respective electronic patient record information packets corresponding to a plurality of potential cases from the electronic patient record storage 331.

Subsequently, an electronic patient record information packet is transmitted from the electronic patient record server 3 to the storage server 2 (Step S27).

Upon receiving the electronic patient record information packet, the storage server 2 in turn sets a file path for the electronic patient record information contained in the packet (Step S28). Thereafter, the storage server 2 stores in the standardized storage 231 the electronic patient record information on the basis of the file path thus set (Step S29). Next, a storage completion notification for notifying that the received electronic patient record information has been stored in the standardized storage 231 is transmitted from the storage server 2 to the electronic patient record server 3 (Step S30).

When the electronic patient record server 3 receives the storage completion notification from the storage server 2, another electronic patient record information packet is transmitted from the electronic patient record server 3 to the storage server 2 (Step S31). Upon receiving the electronic patient record information packet, the storage server 2 in turn sets a file path corresponding thereto (Step S32) and stores the electronic patient record information in the standardized storage 231 on the basis of the file path (Step S33). Subsequently, a storage completion notification is transmitted from the storage server 2 to the electronic patient record server 3 (Step S34). Hereafter, the processing of Steps S31 to S34 is executed repeatedly until all the electronic patient record information packets generated by the electronic patient record server 3 have been transmitted.

Thereafter, when the electronic patient record server 3 determines that all the electronic patient record information packets converted to a format adapted to HL7 have been transmitted (Step S35), a transmission termination notification notifying the termination of the transmission of the electronic patient record information is transmitted from the electronic patient record server 3 to the storage server 2 (Step S36). Upon receiving the transmission termination notification, the storage server 2 in turn shuts off the connection with the electronic patient record server 3 (Step S37).

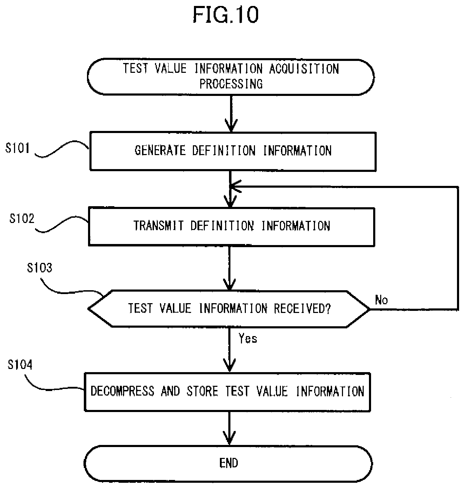

Next, the test value information acquisition processing executed by the terminal device 1 according to the present embodiment will be described with reference to FIG. 10. This test value information acquisition processing is started upon an operation by the user of the terminal device 1 for executing the test value information acquisition processing.

First, the definition information generator 111 generates definition information from a combination of search keys inputted to the terminal device 1 via the user interface 104 by the user for extracting the test value information of the test conducted on a patient who suffers a specific disease (Step S101). At this time, the definition information generator 111 stores the generated definition information in the definition information memory 131 with definition information-identification information added to the definition information.

Next, the test value information acquirer 112 transmits the definition information to the storage server 2 (Step S102). Subsequently, the test value information acquirer 112 determines whether or not test value information has been received within a predefined waiting time after the transmission of the definition information (Step S103). When no test value information has been received within the waiting time (Step S103: No), the test value information acquirer 112 transmits the definition information to the storage server 2 again (Step S102). When the test value information acquirer 112 determines that test value information has been received (Step S103: Yes), the test value information acquirer 112 decompresses the received test value information and stores the decompressed test value information in the main memory 102 (Step S104).

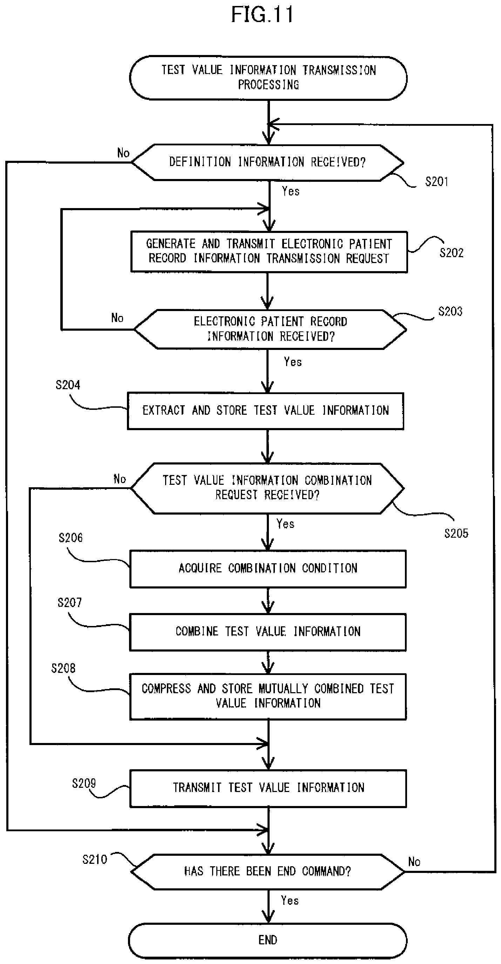

Next, the test value information transmission processing executed by the test value information acquisition server 5 according to the present embodiment will be described with reference to FIG. 11. This test value information transmission processing is started upon turning on the storage server 2.

First, the potential case acquirer 514 determines whether or not definition information has been received from the terminal device 1 (Step S201). When the potential case acquirer 514 determines that no definition information has been received (Step S201: No), the processing at Step S210, which will be described later, is executed.

When the potential case acquirer 514 determines that definition information has been received (Step S201: Yes), the potential case acquirer 514 generates an electronic patient record information transmission request that contains the received definition information and transmits the generated transmission request to the storage server 2 (Step S202). The potential case acquirer 514 then determines whether or not electronic patient record information has been received from the storage server 2 (Step S203). When the potential case acquirer 514 determines that no electronic patient record information has been received from the storage server 2 (Step S203: No), the potential case acquirer 514 executes the processing at Step S202 again. When the potential case acquirer 514 determines that electronic patient record information has been received from the storage server 2 (Step S203: Yes), the potential case acquirer 514 stores the received electronic patient record information of the potential case in the main memory 102. The test value information extractor 515 then extracts test value information from the electronic patient record information acquired from the storage server 2 and stores the extracted test value information in the test value information memory 532 (Step S204).

Subsequently, the combiner 516 determines whether or not a test value information combination request as described above (Step S205) has been received. The combiner 516 receives a test value information combination request when the user has conducted an operation on the storage server 2 for combining a plurality of pieces of test value information, as described above. When the combiner 516 determines that no test value information combination request has been received (Step S205: No), the processing at Step S209, which will be described later, is executed.

When the combiner 516 determines that a test value information combination request has been received (Step S205: Yes), the combiner 516 acquires a combination condition contained in the test value information combination request (Step S206) and combines a plurality of pieces of test value information in accordance with the combination condition (Step S207). Thereafter, the combiner 516 compresses the mutually combined pieces of test value information and stores the compressed information in the test value information memory 532 (Step S208).

Next, the test value information transmitter 517 transmits the compressed test value information stored in the test value information memory 532 to the terminal device 1 (Step S209). Subsequently, the potential case acquirer 514 determines whether or not there has been an end command with which the user commands via the user interface 504 the termination of the test value information transmission processing (Step S210). When the potential case acquirer 514 determines that there has been an end command (Step S210: Yes), the test value information transmission processing is terminated. When the potential case acquirer 514 determines that there has been no end command (Step S210: No), potential case acquirer 514 executes the processing at Step S201 again.

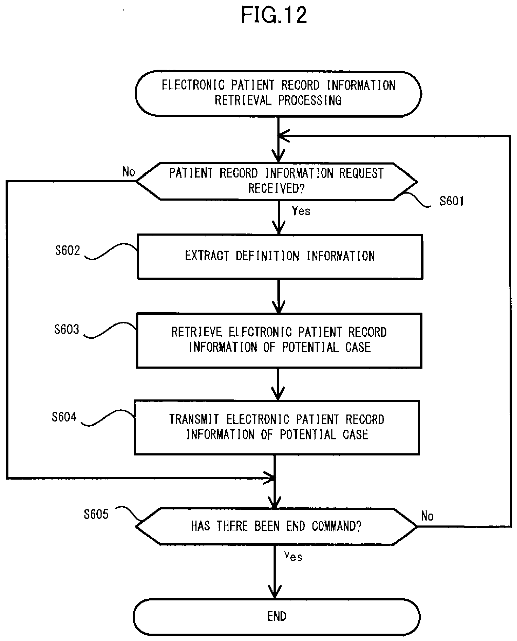

Next, the electronic patient record information retrieval processing executed by the storage server 2 according to the present embodiment will be described with reference to FIG. 12. This electronic patient record information retrieval processing is started upon turning on the storage server 2. First, the receiver 211 determines whether or not an electronic patient record information request has been received from the test value information acquisition server 5 (Step S601). When the receiver 211 determines that no electronic patient record information request has been received (Step S601: No), the processing at Step S605, which will be described later, is executed.

When the receiver 211 determines that an electronic patient record information request has been received (Step S601: Yes), the potential case retriever 214 extracts definition information contained in the received electronic patient record information request (Step S602). Next, the potential case retriever 214 retrieves electronic patient record information of the potential case by using the combination of search keys defined by the extracted definition information (Step S603).

Subsequently, the potential case transmitter 215 transmits the electronic patient record information that the potential case retriever 214 has searched for and acquired to the test value information acquisition server 5 (Step S604).

Thereafter, the receiver 211 determines whether or not there has been an end command with which the user commands via the user interface 204 the termination of the electronic patient record information retrieval processing (Step S605). When the receiver 211 determines that there has been an end command (Step S605: Yes), the electronic patient record information retrieval processing is terminated. When the receiver 211 determines that there has been no end command (Step S605: No), the receiver 211 executes the processing at Step S601 again.

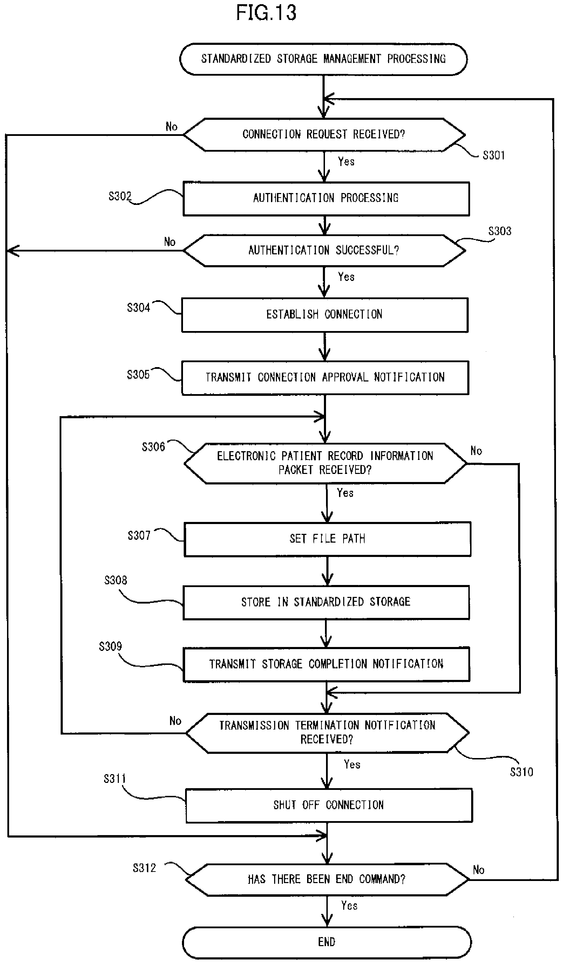

Next, the standardized storage management processing executed by the storage server 2 according to the present embodiment will be described with reference to FIG. 13. This standardized storage management processing is executed in parallel to the above-described electronic patient record information retrieval processing and started upon turning on the storage server 2.

First, the receiver 211 determines whether or not a connection request has been received from the electronic patient record server 3 (Step S301). When the receiver 211 determines that no connection request has been received (Step S301: No), the processing at Step S312, which will be described later, is executed.

When the receiver 211 determines that a connection request has been received (Step S301: Yes), the connection controller 218 executes the authentication processing for the electronic patient record server 3, which is the transmission origin of the connection request (Step S302). When the connection controller 218 fails in the authentication of the electronic patient record server 3 (Step S303: No), the processing at Step S312, which will be described later, is executed.

When the connection controller 218 succeeds in the authentication of the electronic patient record server 3 (Step S303: Yes), the connection controller 218 establishes a connection with the electronic patient record server 3 (Step S304) and transmits a connection approval notification to the electronic patient record server 3 (Step S305).

Next, the analyzer 212 determines whether or not an electronic patient record information packet has been received from the electronic patient record server 3 within a predefined waiting time after the transmission of the connection approval notification (Step S306). When the analyzer 212 determines that no electronic patient record information packet has been received within the waiting time (Step S306: No), the processing at Step S310, which will be described later, is executed.

When the analyzer 212 determines that an electronic patient record information packet has been received within the waiting time (Step S306: Yes), the analyzer 212 analyzes the electronic patient record information packet received by the receiver 211 and extracts the patient identification information, the information indicating the date of consultation, and the information indicating the data type contained in the electronic patient record information packet. The information manager 213 then sets a file path for the acquired electronic patient record information on the basis of the patient identification information, the information indicating the date of consultation, and the information indicating the data type contained in the electronic patient record information (Step S307).

Subsequently, the information manager 213 stores the electronic patient record information in the standardized storage 231 on the basis of the file path thus set (Step S308). Thereafter, the information manager 213 transmits to the electronic patient record server 3 a storage completion notification notifying the completion of the storing of the electronic patient record information in the standardized storage 231 (Step S309).

Next, the receiver 211 determines whether or not a transmission termination notification has been received from the electronic patient record server 3 (Step S310). When the receiver 211 determines that no transmission termination notification has been received (Step S310: No), the processing at Step S306 is executed again.

When the receiver 211 determines that a transmission termination notification has been received (Step S310: Yes), the connection controller 218 shuts off the connection with the electronic patient record server 3 (Step S311). Subsequently, the receiver 211 determines whether or not there has been an end command with which the user commands via the user interface 204 the termination of the standardized storage management processing (Step S312). When the receiver 211 determines that there has been an end command (Step S312: Yes), the standardized storage management processing is terminated. When the receiver 211 determines that there has been no end command (Step S312: No), the receiver 211 executes the processing at Step S301.

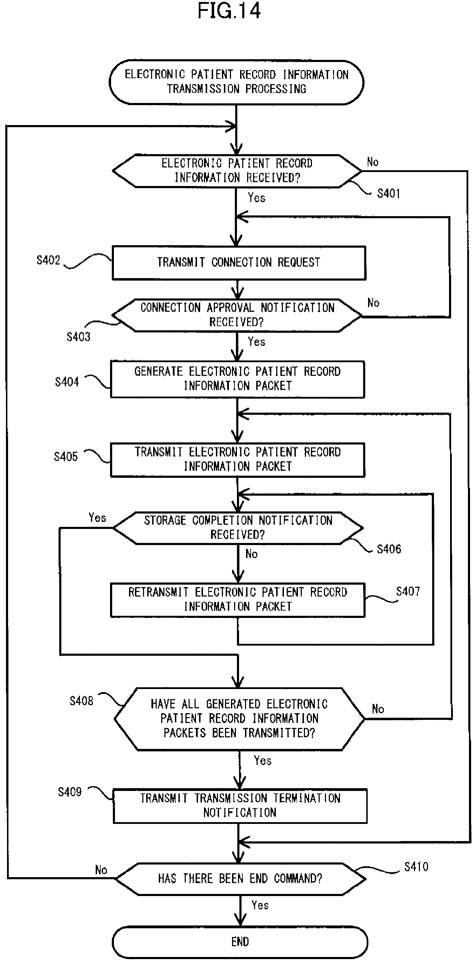

Next, the electronic patient record information transmission processing executed by the electronic patient record server 3 according to the present embodiment will be described with reference to FIG. 14. The electronic patient record information transmission processing is started upon turning on the electronic patient record server 3.

First, the acquirer 311 determines whether or not electronic patient record information has been received from the terminal device 4 (Step S401). When the acquirer 311 determines that no electronic patient record information has been received (Step S401: No), the processing at Step S410, which will be described later, is executed.

When the acquirer 311 determines that electronic patient record information has been received (Step S401: Yes), the transmitter 313 transmits a connection request to the storage server 2 (Step S402). Next, the packet generator 312 determines whether or not a connection approval notification has been received from the storage server 2 within a predefined waiting time after the transmission of the connection request (Step S403). When the packet generator 312 determines that no connection approval notification has been received within the waiting time (Step S303: No), the processing at Step S402 is executed again.

When the packet generator 312 determines that a connection approval notification has been received within the waiting time (Step S403: Yes), the packet generator 312 acquires from the electronic patient record storage 331 a plurality of pieces of electronic patient record information and generates an electronic patient record information packet containing the plurality of acquired pieces of electronic patient record information (Step S404). Subsequently, transmitter 313 transmits the generated electronic patient record information packet to the storage server 2 (Step S405).

Thereafter, the transmitter 313 determines whether or not a storage completion notification has been received from the storage server 2 within a predefined waiting time after the transmission of the electronic patient record information (Step S406). When no storage completion notification has been received within the waiting time (Step S406: No), the transmitter 313 retransmits the same electronic patient record information packet to the storage server 2 (Step S407) and executes the processing at Step S406 again.

When a storage completion notification has been received within the waiting time (Step S406: Yes), the transmitter 313 determines whether or not all the generated electronic patient record information packets have been transmitted to the storage server 2 (Step S408). When the transmitter 313 determines that there is an electronic patient record information packet that has not been transmitted among the plurality of generated electronic patient record information packets (Step S408: No), the transmitter 313 executes the processing at Step S405 again.

When the transmitter 313 determines that all the plurality of generated electronic patient record information packets have been transmitted to the storage server 2 (Step S408: Yes), the transmitter 313 transmits a transmission termination notification to the storage server 2 (Step S409). Next, the acquirer 311 determines whether or not there has been an end command with which the user commands via the user interface 304 the termination of the electronic patient record information transmission processing (Step S410). When the acquirer 311 determines that there has been an end command (Step S410: Yes), the electronic patient record information transmission processing is terminated. When the acquirer 311 determines that there has been no end command (Step S410: No), the acquirer 311 executes the processing at Step S401 again.

Incidentally, when electronic patient record information accumulated in a plurality of electronic patient record servers 3 provided by vendors different from each other is collected, the pieces of electronic patient record information accumulated respectively in the plurality of electronic patient record server 3 may not be compatible with each other. In such a case, the user may be compelled, with respect to test value information contained in respective pieces of electronic patient record information accumulated in the plurality of electronic patient record servers 3, to check the electronic patient record information in each electronic patient record server 3 and read the test value information contained in the electronic patient record information and to store the test value information thus read in a non-transitory recording medium.

In contrast, in the medical information management system according to the present embodiment, the standardized storage 231 of the storage server 2 stores, in a single format, electronic patient record information that is stored in a plurality of electronic patient record servers 3 storing electronic patient record information in different formats. Further, the potential case retriever 214 acquires, on the basis of the definition information, a piece of electronic patient record information on a potential case from among the plurality of pieces of electronic patient record information. The test value information extractor 515 extracts test value information of the potential case from the electronic patient record information acquired by the potential case retriever 214. This allows the collection of the test value information simply by accessing the standardized storage 231 of the storage server 2 as if separately collecting, from the electronic patient record information accumulated in each of the plurality of electronic patient record servers 3, the test value information contained in the electronic patient record information. It is therefore possible to collect efficiently the test value information contained in the electronic patient record information accumulated in the above-described plurality of electronic patient record servers 3. Further, there are other advantages such as, for example, the possibility of facilitating the promotion of secondary uses of the collected test value information.

Further, the test value information acquisition server 5 according to the present embodiment includes a combiner 516 that combines a plurality of pieces of test value information in accordance with a combination condition stipulated by the user. This is advantageous in that the user is allowed to integrate the electronic patient record information of potential cases in accordance with the combination condition stipulated by the user him/herself and it is easier to manage the electronic patient record information.

Further, the standardized storage 231 according to the present embodiment manages a plurality of pieces of electronic patient record information in a directory structure according to the patient identification information, the information indicating the date of consultation, and the information indicating the data type contained in each of the plurality of pieces of electronic patient record information. Further, when the information manager 213 acquires a piece of electronic patient record information, the information manager 213 sets a file path for the acquired piece of electronic patient record information on the basis of the patient identification information, the information indicating the date of consultation, and the information indicating the data type contained in the acquired piece of electronic patient record information. This makes it easier to identify the file path of the electronic patient record information stored in the standardized storage 231 on the basis of the patient identification information, the information indicating the date of consultation, and the like contained in the electronic patient record information and presents an advantage of improved efficiency in retrieving electronic patient record information.

An embodiment of the present disclosure has been described above but the present disclosure is not limited to the above-described configuration of the embodiment, For example, the network NT1 may be disconnected with the network NT2 and the transfer of definition information from the terminal device 1 to the storage server 2 and the transfer of test value information from the storage server 2 to the terminal device 1 may be conducted by means of a non-transitory recording medium such as a universal serial bus (USB) memory.

A medical information management system according to the embodiment may include a function to evaluate the adequacy of the definition information used for extracting a specific piece of medical information from the electronic patient record information accumulated in the storage server 2. A medical information management system according to this modified example may calculate the positive predictive value (PPV) of the medical information extracted by using definition information as described above, by comparing the extracted medical information with an information source regarded as gold standard. Further, the medical information management system may evaluate the adequacy of a combination of search keys more highly when the combination has a higher positive predictive value.

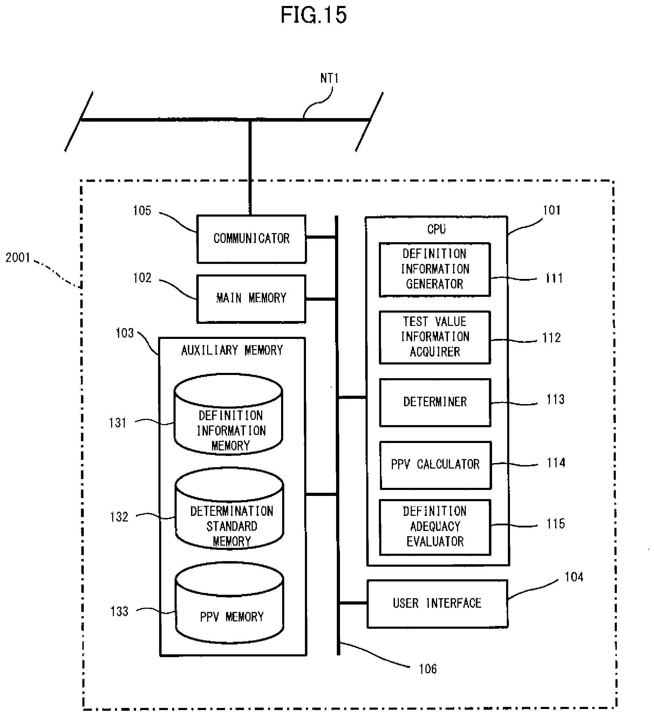

Similarly to the terminal device 1 according to the embodiment, the terminal device 2001 according to the present modified example includes a central processing unit (CPU) 101, a main memory 102, an auxiliary memory 103, a user interface 104, a communicator 105, and a bus 106 that connects these mutually, as illustrated in FIG. 15. Note that in FIG. 15, the features similar to those of the terminal device 1 according to the embodiment are denoted by the same reference numbers as in FIG. 2.

The CPU 101 functions as a definition information generator 111, a test value information acquirer 112, a determiner 113, a PPV calculator (positive predictive value calculator) 114, and a definition adequacy evaluator 115 by executing a program stored in the auxiliary memory 103. Further, the auxiliary memory 103 includes a definition information memory 131, a determination standard memory 132, and a PPV memory 133.

The determination standard memory 132 stores various information necessary for executing the determination method considered to be the best (gold standard) among the determination standards available today for determining the presence or absence of a disease on the basis of test value information. The determination standards are selected by referring to, for example, "Evidence-Based Clinical Practice Guideline for Diabetes 2010", "Atherosclerotic Diseases Prevention Guideline 2007", and the like. The determination standard memory 132 stores a table on which pieces of determination standard value information that indicate determination standard values are associated with corresponding clinical test items and with names of diseases, as illustrated in 16A, for example. The PPV memory 133 stores PPVs of the pieces of test value information acquired by using pieces of definition information, in association with the corresponding pieces of definition information-identification information. Further, the determination standard memory 132 also stores target period information indicating the target period and determination method information indicating the method of determination on whether or not the patient as to whom the determination is made is a true patient.

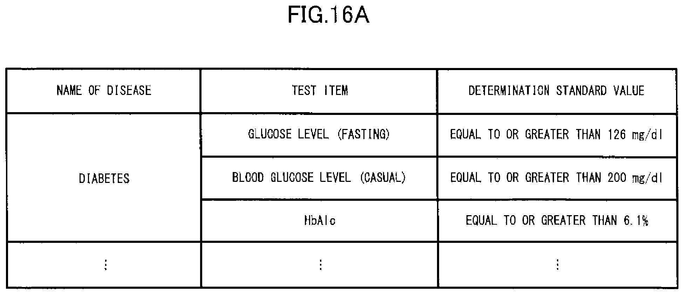

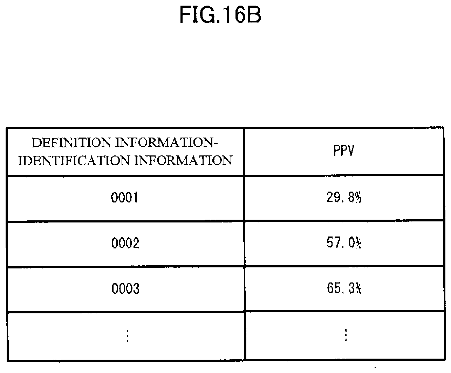

The PPV memory 133 stores a table on which PPVs are associated with pieces of definition information-identification information as illustrated in FIG. 16B, for example.

Referring to FIG. 15 again, the determiner 113 determines whether or not a patient associated with a potential case is positive by referring to the table that contains information indicating the determination standard and is stored in the determination standard memory 132 and by using the test value information acquired by the test value information acquirer 112. Hereinafter, a case in which the patient associated with the potential case is positive will be referred to as a "true case". The determiner 113 determines that a potential case corresponding to test value information is a true case when, for example, the test value information indicates that both of the test values obtained in two tests conducted on one day or different days during the target period are equal to or greater than the determination standard value.

The PPV calculator 114 calculates the positive predictive value (to be referred to as PPV, hereinafter) with respect to the patients associated with the potential cases on the basis of the determination by the determiner 113. More specifically, of all the patients associated with the potential cases, the PPV calculator 114 calculates the number of patients who have been determined to be true cases by the determiner 113 and the number of patients who have not been determined to be true cases. The PPV calculator 114 then calculates the PPV by dividing the number of patients who have been determined to be true cases by the number of all the patients associated with the potential cases. The PPV calculator 114 stores the calculated PPV in the PPV memory 133 in association with the definition information-identification information.

By referring to the PPV memory 133, the definition adequacy evaluator 115 evaluates the adequacy of each piece of definition information on the basis of the PPV with respect to the patients associated with the potential cases. The definition adequacy evaluator 115 executes a ranking operation on pieces of definition information in such a manner that a piece of definition information having a greater PPV is ranked higher in adequacy.

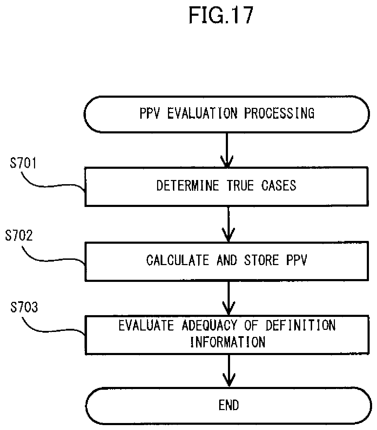

Next, the PPV evaluation processing executed by the terminal device 2001 according to the present modified example will be described with reference to FIG. 17. This PPV evaluation processing is started upon an operation by the user of terminal device 2001 for executing the PPV evaluation processing.

First, the determiner 113 determines whether or not the patients associated with the potential cases are true cases by referring to the determination standard value information, the target period information, and the determination method information stored in the determination standard memory 132 and by using the test value information stored in the main memory 102 (Step S701). Here, the determiner 113 determines with respect to all the patients associated with the potential cases whether or not each case is a true case and stores the results of determination in the main memory 102.

Next, with respect to all the patients associated with the potential cases, the PPV calculator 114 calculates the number of patients who have been determined to be true cases by the determiner 113 and the number of patients who have not been determined to be true cases. The PPV calculator 114 then calculates the PPV by dividing the number of patients who have been determined to be true cases by the number of all the patients associated with the potential cases (Step S702). The PPV calculator 114 stores the calculated PPV in the PPV memory 133 in association with the definition information-identification information.

Subsequently, the definition adequacy evaluator 115 evaluates the adequacy of each piece of definition information by referring to the PPV memory 133 (Step S703).

According to the present configuration, the determiner 113 determines whether or not the patients associated with specific cases are positive by using the test value information extracted by the test value information extractor 515 of the test value information acquisition server 5 and the PPV calculator 114 calculates the PPV evaluation processing with respect to the patients associated with the specific cases on the basis of the results of determination by the determiner 113. The definition adequacy evaluator 115 then evaluates the adequacy of each piece of definition information on the basis of the PPV with respect to the patients associated with the specific cases. This allows the user to select and modify the definition information as appropriate by referring to the result of evaluation of the definition information that defines a combination of search keys. The user thus has a high chance of acquiring pieces of electronic patient record information of positive patients from a plurality of pieces of electronic patient record information by using an appropriate combination of search keys, which presents an advantage of improving the precision of a clinical trial that uses electronic patient record information.

The embodiment has been described with an example in which the clinical information is test value information that indicates results of medical tests but the clinical information is not limited to this and may be, for example, other information that relates to all clinical practices (general condition of the patient, local condition, drug sensitivity, presence or absence of recurrence, survival status, and the like).

The embodiment has been described with an example in which the storage server 2 includes a potential case retriever 214 and a potential case transmitter 215 but the configuration is not limited to this. For example, a server other than the storage server 2, such as the test value information acquisition server 5, may include a potential case retriever 214 and a potential case transmitter 215.

The embodiment has been described with an example in which some of a plurality of electronic patient record servers 3 are connected to the network NT2 while the others of the electronic patient record servers 3 are connected to the network NT4. However, the configuration is not limited to this and, for example, a plurality of electronic patient record servers 3 may be respectively connected to different networks. Alternatively, a plurality of electronic patient record servers 3 may be connected to the same network. Further, a non-transitory recording medium such as a USB memory may be used for the transfer of electronic patient record information between the storage server 2 and a plurality of electronic patient record servers 3.

Further, the functions of the terminal device 1, the storage server 2, and the electronic patient record servers 3 according to the present disclosure may be realized not by a dedicated system but a general-purpose computer system. For example, the terminal device 1, the storage server 2, and the electronic patient record servers 3 that execute the above-described processing may be realized by distributing, to computers connected to a network, a program stored in a non-transitory recording medium (CD-ROM, magnetic disk, or the like) readable by the computer system for executing the above-described functions and by installing the program to the computer system.

Further, the program can be supplied to the computer in any way. For example, the program may be uploaded onto a bulletin board system (BBS) on communication lines and distributed to computers through communication lines. The computers then start the program and execute the program under the control of an OS like other applications. This allows the computers to function as the terminal device 1, the storage server 2, and the electronic patient record servers 3 that execute the above-described functions.

Further, in the embodiment of the present disclosure, the electronic patient record information storage used in the present disclosure is described as a standardized storage but may be other than the standardized storage (for example, extended storage).

Embodiments and modified examples of the present disclosure have been described above (these include those described as additional explanation and the same applies hereinafter) but the present disclosure is not limited to these. The present disclosure encompasses any combination of any of the embodiments and modified examples as appropriate and any modification applied thereto as appropriate.

This application claims the benefit of Japanese Patent Application No. 2017-026368, filed on February 15, 2017, the entire disclosure of which is incorporated by reference herein.

### Industrial Applicability

The present disclosure is suitable for a secondary use of electronic patient record information in pharmaceutical epidemiological studies and the like.

### Reference Signs List

- 1, 2, 4, 2001: Terminal device
- 2: Storage server
- 3: Electronic patient record server
- 5: Test value information acquisition server
- 61, 62: Firewall device
- 101, 201, 301, 501: CPU
- 102, 202, 302, 502: Main memory
- 103, 203, 303, 503: Auxiliary memory
- 104, 204, 304, 504: User interface
- 105, 205, 305, 505: Communicator
- 106, 206, 306, 506: Bus
- 111: Definition information generator
- 112: Test value information acquirer
- 113: Determiner
- 114: PPV calculator
- 115: Definition adequacy evaluator
- 131: Definition information memory
- 132: Determination standard memory
- 133: PPV memory
- 204a: Operation screen
- 211: Receiver
- 212: Analyzer
- 213: Information manager
- 214: Potential case retriever
- 215: Potential case transmitter
- 218: Connection controller
- 231: Standardized storage
- 311: Acquirer
- 312: Packet generator
- 313: Transmitter
- 331: Electronic patient record storage
- 514: Potential case acquirer
- 515: Test value information extractor
- 516: Combiner
- 517: Test value information transmitter
- 532: Test value information memory
- NT1, NT2, NT4: Network
- NT3: Wide area network

## Claims

1. A medical information management system comprising:
an electronic patient record information storage that stores a plurality of pieces of electronic patient record information in a single format, the plurality of pieces of electronic patient record information being stored in a plurality of electronic patient record servers storing the plurality of pieces of electronic patient record information in different formats;
a definition information generator that generates definition information that defines a combination of search keys for retrieving a piece of electronic patient record information on a specific case from among the plurality of pieces of electronic patient record information;
an electronic patient record information retriever that acquires, based on the definition information, a piece of electronic patient record information on the specific case from among the plurality of pieces of electronic patient record information; and
a clinical information extractor that extracts a piece of clinical information of a patient associated with the specific case from the piece of electronic patient record information acquired by the electronic patient record information retriever.

2. The medical information management system according to claim 1, further comprising a combiner that combines a plurality of pieces of the clinical information in accordance with a combination condition stipulated by a user.

3. The medical information management system according to claim 1 or 2, wherein the electronic patient record information storage manages the plurality of pieces of electronic patient record information in a directory structure, according to identification information contained in each of the plurality of pieces of electronic patient record information, and
the medical information management system further comprises an information manager that, upon acquiring a piece of electronic patient record information, sets a file path for the acquired piece of electronic patient record information, based on identification information contained in the acquired piece of electronic patient record information.

4. The medical information management system according to any one of claims 1 to 3, further comprising:
a determiner that determines whether or not the patients associated with the specific cases are positive by using the piece of clinical information extracted by the clinical information extractor;
a positive predictive value calculator that calculates a positive predictive value with respect to the patients associated with the specific cases, based on results of determination by the determiner; and
an adequacy evaluator that evaluates an adequacy of the definition information, based on the positive predictive value with respect to the patients associated with the specific cases.

5. A clinical information acquisition server comprising
a clinical information extractor that extracts a piece of clinical information of a patient associated with a specific case from a piece of electronic patient record information on the specific case acquired from among a plurality of pieces of electronic patient record information, based on definition information that defines a combination of search keys for retrieving the piece of electronic patient record information on the specific case from among the plurality of pieces of electronic patient record information that is stored in an electronic patient record information storage.

6. A medical information management method using an electronic patient record information storage that stores a plurality of pieces of electronic patient record information in a single format, the plurality of pieces of electronic patient record information being stored in a plurality of electronic patient record servers storing the plurality of pieces of electronic patient record information in different formats, the method comprising the steps of:
generating definition information that defines a combination of search keys for retrieving a piece of electronic patient record information on a specific case from among the plurality of pieces of electronic patient record information;
acquiring, based on the definition information, a piece of electronic patient record information on the specific case from among the plurality of pieces of electronic patient record information; and
extracting a piece of clinical information of a patient associated with the specific case from the acquired piece of electronic patient record information.

7. A program for causing a computer to function as:
an electronic patient record information storage that stores a plurality of pieces of electronic patient record information in a single format, the plurality of pieces of electronic patient record information being stored in a plurality of electronic patient record servers storing the plurality of pieces of electronic patient record information in different formats;
a definition information generator that generates definition information that defines a combination of search keys for retrieving a piece of electronic patient record information on a specific case from among the plurality of pieces of electronic patient record information;
an electronic patient record information retriever that acquires, based on the definition information, a piece of electronic patient record information on the specific case from among the plurality of pieces of electronic patient record information; and
a clinical information extractor that extracts a piece of clinical information of a patient associated with the specific case from the piece of electronic patient record information acquired by the electronic patient record information retriever.
